# EUROPEAN PATENT APPLICATION

(11) **EP 4 807 750 A1**
(43) Date of publication of application: **16.09.2026**
(21) Application number: 25162708.9
(22) Date of filing: 10.03.2025
(51) Int. Cl.: G16B 15/00, G16B 30/00

(54) **METHODS FOR IDENTIFYING THE LINKAGES BETWEEN NUCLEOSOMES BY DNA LINKERS, THE PATH OF DNA ALONG NUCLEOSOMES, THE NUCLEOSOME DISTANCE PATTERN OF A PATCH OF CHROMATIN AND THE GENOMIC POSITION THEREOF AND FOR DETERMINING THE NUCLEOTIDE SEQUENCE OF DNA**

(71) Applicant: Max-Planck-Gesellschaft zur Förderung der Wissenschaften e.V., 80539 München (DE)
(72) Inventor: KREYSING, Jan Philipp, 63225 Langen (DE); BECK, Martin, 60438 Frankfurt am Main (DE); CRUZ-LEON, Sergio, 60385 Frankfurt am Main (DE); HUMMER, Gerhard, 61440 Oberursel (DE)
(74) Representative: Kador & Partner Part mbB

(57) **Abstract**

The present invention relates to a method for identifying the linkages between nucleosomes by DNA linkers in a cluster of nucleosomes and to a method for identifying the path of DNA along nucleosomes.

The invention further relates to a method for determining the nucleosome distance pattern of a patch of chromatin and to a method for identifying the genomic position of a patch of chromatin.

Furthermore, the invention relates to a method for determining the nucleotide sequence of DNA.

Furthermore, the invention relates to a computer program, a data processing system, and a computer-readable medium for carrying out the steps of the methods according to the invention.

## Description

The present invention relates to a method for identifying the linkages between nucleosomes by DNA linkers in a cluster of nucleosomes and to a method for identifying the path of DNA along nucleosomes.

The invention further relates to a method for determining the nucleosome distance pattern of a patch of chromatin and to a method for identifying the genomic position of a patch of chromatin.

Furthermore, the invention relates to a method for determining the nucleotide sequence of DNA.

Furthermore, the invention relates to a computer program, a data processing system, and a computer-readable medium for carrying out the steps of the methods according to the invention.

The genetic material within a cell consists of DNA that needs to be packaged into a nucleus. One mechanism for packing DNA is by wrapping DNA around histone proteins. The resulting protein-DNA complexes form nucleosomes, often depicted as "beads on a string", which in turn fold into stable, higher-order structures, to finally form the complex three-dimensional arrangement known as chromatin. The hierarchical architecture of chromatin has been studied extensively. Light microscopy imaging and advanced sequencing techniques have revealed chromosome organization, chromatin compartments or topologically associating domains (TADs), while structural biology techniques have been used to analyze the molecular details of how DNA is wrapped around individual reconstituted nucleosomes (T. Misteli, The Self-Organizing Genome: Principles of Genome Architecture and Function. Cell 183, 28-45 (2020).). However, how chromatin is organized at the level of multiple nucleosomes inside of cells, in particular how nucleosomes are arranged with respect to each other in native chromatin, remains the subject of active research.

Nucleosomes are formed by a C2-symmetric octamer of two copies of each of the core histones H2A, H2B, H3 and H4 and 147 base pairs (bp) of DNA that is wrapped around the histone octamer in a left-handed superhelical turn. Nucleosomes are flanked by stiff DNA linkers of variable length that project towards the next nucleosome (K. Luger, A. W. Mäder, R. K. Richmond, D. F. Sargent, T. J. Richmond, Crystal structure of the nucleosome core particle at 2.8 A resolution. Nature 389, 251-260 (1997)). A single H1 linker histone can bind to the DNA of the core nucleosome superhelix thereby also interact with the ends of the two adjacent DNA linkers, called DNA linker arms. The resulting structure disrupts the nucleosome's 2-fold symmetry, and is consequently asymetric (J. Bednar, I. Garcia-Saez, R. Boopathi, A. R. Cutter, G. Papai, A. Reymer, S. H. Syed, I. N. Lone, O. Tonchev, C. Crucifix, H. Menoni, C. Papin, D. A. Skoufias, H. Kurumizaka, R. Lavery, A. Hamiche, J. J. Hayes, P. Schultz, D. Angelov, C. Petosa, S. Dimitrov, Structure and Dynamics of a 197 bp Nucleosome in Complex with Linker Histone H1. Mol Cell 66, 384-397.e8 (2017)). The core nucleosome with H1 bound and its two DNA linkers, is referred to as a chromatosome, is thought to be a major building block of higher-order chromatin structures. H1 thought to bind to most nucleosomes, but H1 binding is dependent on cell types and chromatin states, i.e. H1 is more abundant in heterochromatin. H1 variants can bind to either of the two DNA linker arms, the DNA wound around the histone octamer, or all three DNA structures simultaneously, resulting in differential stabilization and either on-dyad or off-dyad arrangements (D. V. Fyodorov, B. R. Zhou, A. I. Skoultchi, Y. Bai, Emerging roles of linker histones in regulating chromatin structure and function. Nat Rev Mol Cell Biol 19, 192-206 (2018); P. Chen, W. Li, G. Li, Structures and Functions of Chromatin Fibers. Annu Rev Biophys 50, 95-116 (2021)).

Local chromatin folding is known to be determined by nucleosome organization (P. Chen, W. Li, G. Li, Structures and Functions of Chromatin Fibers. Annu Rev Biophys 50, 95-116 (2021)). Several models have been proposed for the arrangement of nucleosomes into a 30 nm chromatin fiber, such as e.g. the zig-zag model in which alternating nucleosomes interact, or the solenoid model that implies interactions of neighboring nucleosomes. Indeed, arrangements of multiple nucleosomes on DNA in vitro are characterized by a zig-zag arrangement, whereby nucleosomes can stack with each other, and the DNA linkers restrain the orientation of consecutive nucleosomes (M. Zhang, C. Diaz-Celis, J. Liu, J. Tao, P. D. Ashby, C. Bustamante, G. Ren, Angle between DNA linker and nucleosome core particle regulates array compaction revealed by individual-particle cryo-electron tomography. Nat Commun 15, 4395 (2024)). In addition, a flat ladder-like structure and twisted arrangements have been described. Chromatin folding and its compaction behavior is influenced by H1 binding in on-dyad or off-dyad position and on the nucleosome repeat lengths (NRLs) (A. Routh, S. Sandin, D. Rhodes, Nucleosome repeat length and linker histone stoichiometry determine chromatin fiber structure. Proc Natl Acad Sci U S A 105, 8872-8877 (2008)). Reduced DNA linker lengths and consequently shorter NRLs sterically hinder H1 binding and result in less regular arrays, which in turn are associated with active chromatin. These molecular details have been derived from extensive in vitro structural analyses of reconstituted or purified histones, often using artificial, so-called 601 nucleosome positioning sequence (NPS) repeats (P. T. Lowary, J. Widom, New DNA sequence rules for high affinity binding to histone octamer and sequence-directed nucleosome positioning. J Mol Biol 276, 19-42 (1998). To which extent these studies reliably recapitulate the architecture of native chromatin in situ, which is not only dependent on NRLs, but also affected by phase separation, ionic strength and various other associated proteins beyond linker H1, remains largely unknown.

Several recent studies have investigated nucleosome arrangements in different cell types in situ using cryo-electron tomography (cryo-ET). Although nucleosome stacking was observed, an overall relatively heterogenous chromatin arrangement was reported (J. K. Chen, T. Liu, S. Cai, W. Ruan, C. T. Ng, J. Shi, U. Surana, L. Gan, Nanoscale analysis of human G1 and metaphase chromatin in situ bioRxiv (2023)). Human cells contained a fibrous chromatin structure lining the nuclear envelope (18, 19), with exclusion zones beneath nuclear pores (B. Wang, R. Kronenberg-Tenga, V. Rosti, E. Di Patrizio Soldateschi, Q. Luo, L. Pinet, M. Eibauer, R. Boujemaa-Paterski, B. Schuler, C. Lanzuolo, O. Medalia, The molecular basis of lamin-specific chromatin interactions bioRxiv (2024)). However, the respective in situ structures of nucleosomes were not resolved to secondary structure, and the topology of neighboring nucleosomes was not assessed with respect to the above-discussed structural features that are known to constitute chromatin architecture in vitro. Methods to determine nucleosome positions in cells in situ based on imaging techniques and to determine their genomic position are presently lacking. The 3D structure of chromatin inside cells and its distinct multi-nucleosome arrangements that is determined by nucleosome linker lengths, remain poorly resolved.

In view of the above, there is a need and accordingly it is the object of the present invention to provide new methods for identifying the linkages between nucleosomes in 3-dimensional space, their connections by DNA linkers, and the path of the DNA along the nucleosomes. It is further the object of the present invention to provide new methods for determining the distance pattern between nucleosome in chromatin, the genomic position and the genomic sequence of the DNA on the nucleosomes and along the intervening linkers.

The instant invention is based on the surprising finding that using the positions and orientations of nucleosomes which may be derived, e.g. for example from imaging data, the linkages between nucleosomes by DNA linkers and the path of DNA along nucleosomes can be identified. It was further found that the length of the DNA given by its path between the pairs of nucleosomes can be calculated. By connecting multiple nucleosomes in this way, the consequent path of DNA along nucleosomes, taken together with these nucleosomes, represents a patch of chromatin.

It was further surprisingly found that a nucleosome distance pattern of a patch of chromatin can be used to identify its genomic position and nucleotide sequence. It was further surprisingly found that imaging data can be used for determining the sequence of DNA.

The present invention therefore provides a method for identifying the linkages between nucleosomes by DNA linkers in a cluster of nucleosomes comprising, or consisting of:
(i) determining the probability of DNA linkers being present between nucleosomes comprising, or consisting of, calculating the bending energy of hypothetical linkages between pairs of nucleosomes using position and orientation data of the nucleosomes,
whereby a lower bending energy is indicating a higher probability.

The present invention further provides a method for determining the nucleosome distance pattern of a patch of chromatin comprising, or consisting of, the steps of:
(i) identifying the path of DNA along nucleosomes, preferably in an image,
(ii) calculating the lengths of the DNA of pairs of nucleosomes of the path, wherein the length of the DNA of a pair of nucleosomes comprises the DNA linker length (L) and the length of the DNA wound up on one nucleosome.
wherein the lengths of DNA between the pairs of nucleosomes of the path defines the nucleosome distance pattern of the patch of chromatin.

Technically, a nucleosome is sometimes seen as the core particle plus DNA linkers; however, the word nucleosome is often synonymous with the core particle alone. A nucleosome within the meaning of the instant invention is the core particle including the histone octamer and DNA wound around it, but without the DNA linkers.

A DNA linker, also termed linker DNA, is double-stranded (ds) DNA of variable length, usually 5-90 base pairs long, in between two nucleosome cores that physically connects the two nucleosome cores together. Linker DNA is seen as the string in the "beads on a string model". Hence, a DNA linker does not comprise the DNA wrapped around a nucleosome core particle.

DNA linker arms are the respective ends of the DNA linkers that transition into the DNA wound around the histone octamer of the nucleosome. DNA linker arms may interact with H1. DNA linker arms emerge from the nucleosome in a specific angle that determine the orientation of the DNA linker and accordingly the orientation of the nucleosomes.

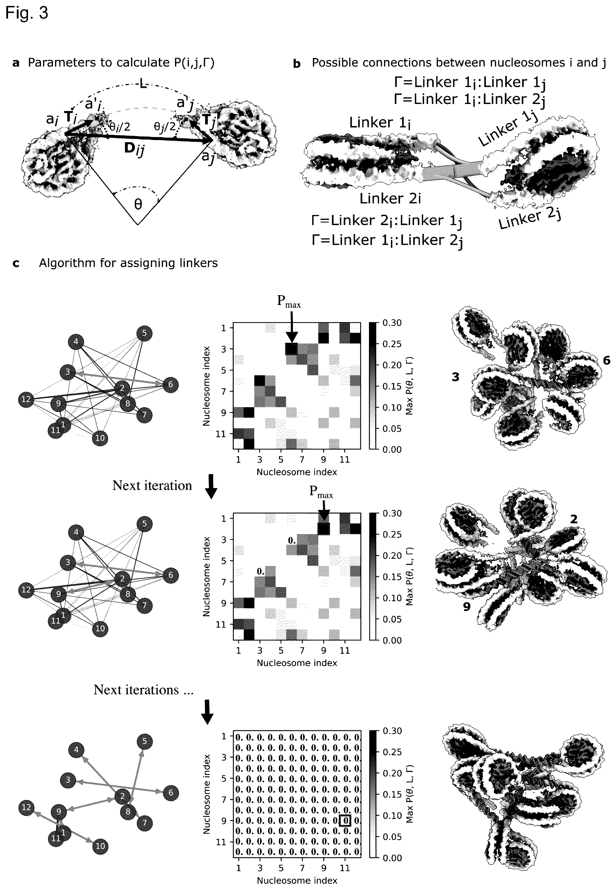

In a cluster of nucleosomes, linkages between each of the nucleosomes present in the cluster are possible in theory. Furthermore, between each pair of nucleosomes, in theory four connections are possible between the two DNA linker arms on each nucleosome. The four hypothetical linkages between a pair of nucleosomes are shown in Fig. 3b. In the present invention, all these linkages are termed hypothetical linkage between nucleosomes.

However, in chromatin the nucleosomes are consecutively connected, such that every nucleosome may be connected to two further nucleosomes by respective DNA linkers, except the 1^{st} and last nucleosome which are only connected to one nucleosome by a DNA linker. In particular, a DNA linker connects to one DNA linker arm of the two linker arms on the 1st nucleosome and to one DNA linker arm of the two linker arms on the 2^{nd} nucleosome. Hence, a pair of nucleosomes is connected by one DNA linker. Accordingly, for a number of N nucleosomes a maximum of N-1 DNA linkers may be present between the nucleosomes.

The bending energy of a hypothetical linkage between the nucleosomes is the energy required for a DNA linker formation between the pair of nucleosomes. The bending energy can be calculated as the energy required to bend a segment of DNA into a DNA linker between a pair of nucleosomes. More particularly, the bending energy can be calculated as the energy required to bend a segment of DNA into a DNA linker between the DNA linker arms on the nucleosomes of a pair of nucleosomes. Accordingly, the lower the bending energy, i.e. the energy required for a DNA linker formation between the pair of nucleosomes, the higher the probability that a DNA linker is present between the pair of nucleosomes. Straighter linkers have lower bending energy and thus higher probability as being present between pairs of nucleosomes.

In a first aspect, the present invention provides a method for identifying the linkages between nucleosomes by DNA linkers in a cluster of nucleosomes comprising, or consisting of:
(i) determining the probability of DNA linkers being present between nucleosomes comprising, or consisting of, calculating the bending energy of hypothetical linkages between pairs of nucleosomes using position and orientation data of the nucleosomes,
whereby a lower bending energy is indicating a higher probability.

Hence, according to the invention the bending energy of hypothetical linkages between pairs of nucleosomes in the cluster is calculated using position and orientation data of the nucleosomes in the cluster. Thereby, the probability of DNA linkers being present between nucleosomes in the cluster is determined.

In other words, determining the probability of DNA linkers being present between nucleosomes is effected comprising, or consisting of, calculating the bending energy of hypothetical linkages between pairs of nucleosomes using position and orientation data of the nucleosomes.

Preferably, the probability of DNA linkers being present between pairs of nucleosomes is determined.

Preferably, the linkages between nucleosomes by DNA linkers in a cluster of nucleosomes are arranged in 3-dimensional space. Hence, preferably, the linkages in 3-dimensional space between nucleosomes by DNA linkers in a cluster of nucleosomes are identified.

Preferably, the position and orientation data of the nucleosomes is the spatial position and spatial orientation data of the nucleosomes in the cluster.

Preferably, the position and orientation data of the nucleosomes in the cluster used in step (i) is provided, preferably in or before step (i).

Preferably, the orientation data of a nucleosome in the cluster is defined by the spatial orientation of its DNA linker arms.

Preferably, the bending energy of a hypothetical linkage between a pair of nucleosomes is calculated and/or the probability of DNA linkers being present between nucleosomes is determined on the basis of a physics-based DNA model.

The simplest physics-based model is based on theory of polymers and estimates the DNA bending energy associated with a particular linkage using the persistence length as a measure of the bending stiffness of a double-stranded DNA polymer. The persistence length of a double-stranded DNA polymer is typically around 50 nm. A more refined model uses the energy associated with bending and torsional twisting of the DNA linker. An even more refined model uses the free energy for the elastic deformation of the DNA linker, as required to connect the two nucleosome linker arms, and calculates this free energy with the help of molecular dynamics simulations using classical mechanical force fields such as the bsc1 force field for DNA (Ivani, I., Dans, P., Noy, A. et al. Parmbsc1: a refined force field for DNA simulations. Nat Methods 13, 55-58 (2016). https://doi.org/10.1038/nmeth.3658).

Preferably, the bending energy of a hypothetical linkage between a pair of nucleosomes is calculated and/or the probability of DNA linkers being present between nucleosomes is determined using the persistence length of a double-stranded DNA polymer.

As detailed above, nucleosomes are connected by double-stranded (ds) DNA linkers. Single-molecule elasticity measurements have shown that the entropic elasticity of dsDNA in aqueous solutions is accurately described by the worm-like chain (WLC) model with a fixed contour length (S. B. Smith, L. Finzi, C. Bustamante, Direct mechanical measurements of the elasticity of single DNA molecules by using magnetic beads. Science 258, 1122-1126 (1992)). WLC models describe the physical behavior of polymers that are semi-flexible: fairly stiff with successive segments pointing in roughly the same direction, and with persistence length within a few orders of magnitude of the polymer length. Deviations of the chain from its straight configuration result in a bending energy (J. F. Marko, Stretching must twist DNA Europhysics Letters (EPL) 38, 183-188 (1997)).

Preferably, for calculating the bending energy of hypothetical linkages between pairs of nucleosomes and/or for determining the probability of DNA linkers being present between nucleosomes, the DNA is described with the WLC model. Preferably, the contour length of the DNA linker is in a predefined and weighted range of contour lengths. The distribution of contour lengths depends on the species, tissue, cell type, cell state, and genome location. The weight of different contour lengths is that of an exponential distribution with this length as the characteristic length. Preferably, the characteristic length of the distribution is 1 nm to 50 nm, more preferably 5 nm to 30 nm, even more preferably 10 nm to 25 nm, most preferably 15 nm.

The bending energy is preferably calculated for a hypothetical linkage between one or more pairs of nucleosomes in the cluster. Preferably, the bending energy is calculated for hypothetical linkages between at least 50%, more preferably at least 70%, even more preferably at least 90% and most preferred for all pairs of nucleosomes in the cluster. Accordingly, the probability of DNA linkers being present between nucleosomes is determined for one or more pairs of nucleosomes in the cluster. Preferably, the probability of DNA linkers being present between nucleosomes is determined for at least 50%, more preferably at least 70%, even more preferably at least 90% and most preferred for all pairs of nucleosomes in the cluster.

Furthermore, the bending energy may be calculated for one, two, three or each of the four hypothetical linkages between a pair of nucleosomes in the cluster. In one preferred embodiment the bending energy of one of the four hypothetical linkages between a pair of nucleosomes is calculated. In a further preferred embodiment, the bending energy of two, three or each of the four hypothetical linkages between a pair of nucleosomes is calculated. Accordingly, the probability of DNA linkers being present between nucleosomes may be determined for one, two, three or each of the four hypothetical linkages between a pair of nucleosomes in the cluster. In one preferred embodiment, the probability of DNA linkers being present between nucleosomes is determined for one of the four hypothetical linkages between a pair of nucleosomes. In a further preferred embodiment, the probability of DNA linkers being present between nucleosomes is determined for two, three or each of the four hypothetical linkages between a pair of nucleosomes.

Preferably, the bending energy of a hypothetical linkage between a pair of nucleosomes is calculated as a function comprising, or consisting of, the DNA linker length (L) and/or the DNA linker bending angle (θ) between the pair of nucleosomes of the hypothetical linkage. More preferably, the bending energy of a hypothetical linkage between a pair of nucleosomes is calculated as a function comprising, or consisting of, the DNA linker length (L) and/or the DNA linker bending angle (θ) between the DNA linker arms on the pair of nucleosomes of the hypothetical linkage. Even more preferably, the bending energy of a hypothetical linkage between a pair of nucleosomes is calculated as a function comprising, or consisting of, the DNA linker length (L) and the DNA linker bending angle (θ), between the pair of nucleosomes, even more preferably between the DNA linker arms on the pair of nucleosomes of the hypothetical linkage. The DNA linker length (L) and the DNA linker bending angle (θ) between the DNA linker arms on the pair of nucleosomes of the hypothetical linkage are shown in Fig. 3a.

Preferably, the bending energy of a hypothetical linkage between a pair of nucleosomes is calculated as a function further comprising the rotational pitch between the pair of nucleosomes of the hypothetical linkage. Thereby the rotational pitch refers to the rotation of the connected nucleosome around the two connected linker arms with respect to each other. Due to the helical nature of B DNA, the linker length (L) and consequently the bending energy and respective probability, can be determined more precisely if the rotational pitch is considered.

The DNA linker length (L) and/or the DNA linker bending angle (θ) are preferably determined using the position and orientation data of the nucleosomes of the hypothetical linkage, more preferably using the position and orientation data of the DNA linker arms on the pair of nucleosomes of the hypothetical linkage.

Preferably, the DNA linker length (L) and the DNA linker bending angle (θ) are determined using the distance between the pair of nucleosomes of the hypothetical linkage. Preferably, the distance between the pair of nucleosomes of the hypothetical linkage is determined using the position and orientation data of the nucleosomes of the hypothetical linkage, more preferably using the position and orientation data of the DNA linker arms on the pair of nucleosomes of the hypothetical linkage.

Preferably the distance between the pair of nucleosomes of the hypothetical linkage is the Euclidian distance. More preferably, the DNA linker length (L) and the DNA linker bending angle (θ) are determined using the distance between the DNA linker arms on the pair of nucleosomes of the hypothetical linkage.

Preferably, the bending energy of a hypothetical linkage between a pair of nucleosomes is calculated according to the following formula (1) ${U}_{bend} \left(θ, L\right) = \frac{2 {l}_{p}}{L} {\left(\frac{θ}{2}\right)}^{2} {k}_{B} T$ wherein (L) is the DNA linker length and θ is the DNA linker bending angle between the pair of nucleosomes of the hypothetical linkage, *lₚ* is the B-DNA persistence length at physiological conditions and *k_{B}* is the Boltzmann constant, *T* is the temperature, and thus *k_{B}T* is the thermal energy.

*lₚ*, the B-DNA persistence length at physiological conditions, is a parameter that can be adjusted according to species, tissue, cell type, cell state, and genome location. Preferably, *lₚ* is 30 nm to 70 nm, more preferably 40 nm to 60 nm, even more preferably 45 nm to 55 nm, most preferably 50 nm. Preferably, *k_{B}T* is 2.27 kJ/mol to 3.10 kJ/mol, more preferably 2.44 kJ/mol to 2.69 kJ/mol, and most preferably 2.58 kJ/mol.

Preferably, the DNA linker length (L) is the arc length between the pair of nucleosomes of the hypothetical linkage. More preferably, the DNA linker length (L) between the pair of nucleosomes of the hypothetical linkage is the length of the arc of a circle sector of radius r and central angle θ determined according to the following formula (2) $L = θr$ and wherein the radius r is related to the Euclidian distance D between two nucleosomes, preferably the ends of the linker arms on the two nucleosomes according to the following formula (3) $r = D / \left(2 sin\left(θ/2\right)\right)$

Preferably, the probability of a DNA linker being present between a pair of nucleosomes is determined comprising, or consisting of, calculating the bending energy of hypothetical linkage between pairs of nucleosomes as detailed above. Accordingly, preferably determining the probability of DNA linkers being present between nucleosomes is effected by calculating the bending energy of hypothetical linkages between pairs of nucleosomes using position and orientation data of the nucleosomes.

More preferably, the probability of a DNA linker being present between a pair of nucleosomes is determined comprising, or consisting of, calculating the bending energy of hypothetical linkage between pairs of nucleosomes as detailed above and further by determining the DNA linker length (L) between the pair of nucleosomes of the hypothetical linkage as detailed above. Accordingly, more preferably, determining the probability of DNA linkers being present between nucleosomes is effected by calculating the bending energy of hypothetical linkages between pairs of nucleosomes using position and orientation data of the nucleosomes and further by determining the DNA linker length (L) between the pair of nucleosomes of the hypothetical linkage as detailed above. More preferably, the probability of a DNA linker being present between a pair of nucleosomes is determined by the bending energy of the hypothetical linkage between the pair of nucleosomes and by the DNA linker length (L) between the pair of nucleosomes of the hypothetical linkage. The DNA linker length (L) between the pair of nucleosomes of the hypothetical linkage may be, preferably is, determined as described above.

The DNA linker length L, as detailed above may, preferably is, used for calculating the bending energy. The advantage of taking the DNA linker length (L) again into account for determining the probability of a DNA linker being present between a pair of nucleosomes is that long linker lengths are penalized. Hence, the resulting probability function penalizes very long but straight as well as very short but strongly bend linkers.

More preferably, the probability of a DNA linker being present between a pair of nucleosomes is determined comprising, or consisting of, the bending energy of the hypothetical linkage between the pair of nucleosomes and further by determining the ratio of the DNA linker length (L) between the two nucleosomes of the hypothetical linkage and a reference length. More preferably, the probability of a DNA linker being present between a pair of nucleosomes is determined by the bending energy of the hypothetical linkage between the pair of nucleosomes and further by the ratio of the DNA linker length (L) between the two nucleosomes of the hypothetical linkage and a reference length.

Even more preferably, the probability of a DNA linker being present between a pair of nucleosomes is determined according to the following formula (4) $P \left(L, θ\right) = {e}^{- \frac{L}{{L}_{0}}} {e}^{- \frac{{U}_{bend} \left(θ, L\right)}{{k}_{B} T}}$ wherein *L*₀ is a reference linker length,(L)is the DNA linker length between the pair of nucleosomes of the hypothetical linkage and *k_{B}T* is the thermal energy. Preferably *L*₀ is, the reference linker length, is a parameter that can be adjusted according to species, tissue, cell type, cell state, and genome location. Preferably, *L*₀ is 5 nm to 30 nm, more preferably 10 nm to 25 nm, most preferably 15 nm. Preferably, *k_{B}T* is within the ranges as defined above.

Preferably, the method further comprises
(ii) defining that DNA linkers are present between pairs of nucleosomes for which the highest probabilities result.

Thereby, it is defined that pairs of nucleosomes are connected by DNA linkers.

In one preferred embodiment, in step (ii) a plurality of DNA linkers with the highest probabilities are defined as present between the respective pairs of nucleosomes. Hence, preferably, a plurality of connections are defined as being present in one attempt. Preferably, for the plurality of DNA linkers with the highest probabilities an overall probability is determined. Such overall probability involves a step of determining whether DNA linkers for which the highest probabilities result are technically sensible. Thereby, it is defined that a plurality of pairs of nucleosomes are connected by DNA linkers.

In a further, more preferred embodiment, in step (ii) the DNA linker with the highest probability is defined as present between the pair of nucleosomes. Then, consecutively further DNA linkers are defined as being present between pairs of nucleosomes with the next-highest probabilities. Hence, preferably connections are defined as being present in a step-wise manner whereby the most likely connection is defined first and the least likely connection is defined last. Preferably, the method is continued until no more linkages between nucleosomes by DNA linkers in the cluster of nucleosomes are possible. For a number of N nucleosomes, a maximum of N-1 DNA linkers may be defined as being present between the nucleosomes. Hence, thereby, it is defined that a pair of nucleosomes is connected by a DNA linker, in particular, a first pair and then subsequent pairs of nucleosomes are connected by a DNA linker.

Preferably, step (ii) comprises a step of determining whether DNA linkers for which the highest probabilities result are technically sensible. Preferably, such determining is conducted before defining that DNA linkers are present between pairs of nucleosomes for which the highest probabilities result. Preferably, such determining is conducted each time before a DNA linker is defined as present between a pair of nucleosomes. Such determining may, e.g. for example, comprise, preferably consist of, setting a predefined probability threshold and/or further excluding technically not sensible DNA linkers. Such technically not sensible DNA linkers, comprise, preferably consist of, multiple connections between two nucleosomes, multiple connections to a linker arm, connection back to the same nucleosome by forming a loop, and/or a closed form of DNA as a loop over multiple nucleosomes.

Accordingly, preferably, in step (ii) of the method, a DNA linker for which the highest probability results, is only defined as being present between a pair of nucleosomes if its probability exceeds a predefined threshold. The advantage of using a predefined threshold is that very unlikely connections between pairs of nucleosomes are not considered. Hence, preferably, DNA linkers for which the highest probabilities result are not defined as DNA linkers present between pairs of nucleosomes in step (ii) if the probability of the DNA linkers is below a preset threshold Pₘᵢₙ. In this case the method is terminated. Pₘᵢₙ is a parameter that can be adjusted according to species, tissue, cell type, cell state, and genome location. Preferably Pₘᵢₙ is 1.0 to 0.01, more preferably 0.05 to 0.5, most preferably Pₘᵢₙ is 0.1.

Furthermore, preferably the DNA linkers for which the highest probabilities result are not defined as DNA linkers present between nucleosomes in step (ii), if the DNA linkers are between pairs of nucleosomes for which pairs of nucleosomes a defined DNA linker is already present. In this case the method is continued with the DNA linkers for which the next highest probabilities result. The advantage thereof is that multiple connections between two nucleosomes are not considered.

Furthermore, preferably the DNA linkers for which the highest probabilities result are not defined as DNA linkers present between nucleosomes in step (ii), if the DNA linkers are between two linker arms on nucleosomes of which one linker arm on a nucleosome is part of an already defined DNA linker present between a pair of nucleosomes. In this case the method is continued with the DNA linkers for which the next highest probabilities result. The advantage thereof is that multiple connections to a linker arm are not considered.

Furthermore, preferably the DNA linkers for which the highest probabilities result are not defined as DNA linkers present between nucleosomes in step (ii), if the DNA linkers are between two DNA linker arms on nucleosomes of which one DNA linker arm on a nucleosome returns to the other DNA linker arm of the same nucleosome. In this case the method is continued with the DNA linkers for which the next highest probabilities result. The advantage thereof is that a connection back to the same nucleosome by forming a loop is not considered.

Furthermore, preferably the DNA linkers for which the highest probabilities result are not defined as DNA linkers present between nucleosomes in step (ii), if the DNA linkers close the already defined DNA linkers to a closed loop of DNA linkers. In this case the method is continued with the DNA linkers for which the next highest probabilities result. The advantage thereof is that a closed form of DNA as a loop over multiple nucleosomes is not considered.

Accordingly, preferably step (ii) comprises
(ii.a) identifying the DNA linker having the highest probability,
(ii.b) defining that said DNA linker is present between the pair nucleosomes, optionally, or preferably, if the probability of the DNA linker is above a preset threshold Pₘᵢₙ, the DNA linker is not between a pair of nucleosomes for which pair of nucleosomes a defined DNA linker is already present, the DNA linker is not between two linker arms on nucleosomes of which one linker arm on a nucleosome is part of an already defined DNA linker present between a pair of nucleosomes, the DNA linker is not between two DNA linker arms on nucleosomes of which one DNA linker arm on a nucleosome returns to the other DNA linker arm of the same nucleosome, and/or the DNA linker does not close the already defined DNA linkers to a closed loop of DNA linkers,
returning to step (ii.a), or, if the probability of the DNA linker is below a preset threshold Pₘᵢₙ. the method is terminated in step (ii.b),

More preferably step (ii) comprises
(ii.a) identifying the DNA linker having the highest probability,
(ii.b) defining that said DNA linker is present between the pair nucleosomes, optionally, or preferably, if the probability of the DNA linker is above a preset threshold Pₘᵢₙ, the DNA linker is not between a pair of nucleosomes for which pair of nucleosomes a defined DNA linker is already present, the DNA linker is not between two linker arms on nucleosomes of which one linker arm on a nucleosome is part of an already defined DNA linker present between a pair of nucleosomes, the DNA linker is not between two DNA linker arms on nucleosomes of which one DNA linker arm on a nucleosome returns to the other DNA linker arm of the same nucleosome, and/or the DNA linker does not close the already defined DNA linkers to a closed loop of DNA linkers,
(ii.c) setting the probability of said defined DNA linker to zero, and
returning to step (ii.a), or, if the probability of the DNA linker is below a preset threshold Pₘᵢₙ. the method is terminated in step (ii.b).

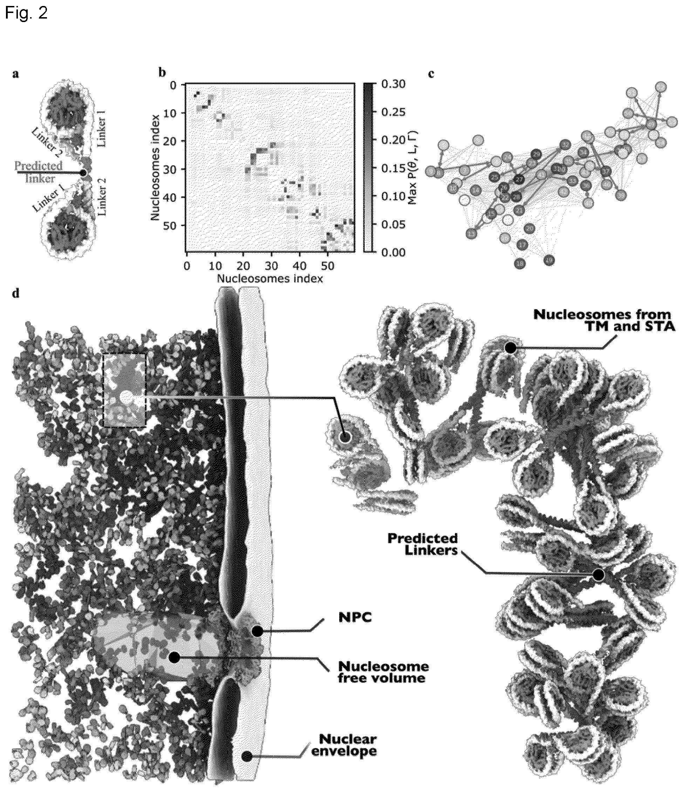

Step ii.a to ii.c are illustrated in Fig. 2b, c and Fig. 3c.

Preferably, the bending energy of a hypothetical DNA linkage between a pair of nucleosomes is calculated in step (i) for each of the four hypothetical DNA linkages between the four DNA linker arms on the pair of nucleosomes as described above. In other words, preferably the bending energy of each of the four hypothetical linkages between a pair of nucleosomes is calculated as described above.

Preferably, the bending energy of a hypothetical DNA linkage between the nucleosomes is calculated in step (i) as a function of the DNA linker length (L) and/or the DNA linker bending angle (θ) as described above for each of the four hypothetical DNA linkages between the four DNA linker arms on a pair of nucleosomes.

Hence, preferably, the probability of a DNA linker being present between a pair of nucleosomes is determined in step (i) for each of the four hypothetical DNA linkages between the four DNA linker arms on the pair of nucleosomes as described above. More preferably, the probability of a DNA linker being present between a pair of nucleosomes is calculated in step (i) for each of the four hypothetical DNA linkages between the four DNA linker arms on the pair of nucleosomes according to the following formula (5) ${P}_{ij} \left(L, θ, Γ\right) = \left({e}^{\frac{- L \left(Γ\right)}{{L}_{0}}}\right) \left({e}^{\frac{- Δ {U}_{bend} \left(θ\left(Γ\right)\right)}{{k}_{B} T}}\right)$
wherein i denotes the linkers of a nucleosome as Linker1ᵢ, and Linker2_{i.}and
┌ denotes for the four possible ways to connect a pair of nucleosomes (i, j), that is: ∈[(Linker1_i:Linker1_j),(Linker1_i:Linker2_j),(Linker2_i:Linker1_j),(Linker2_i Linke r2_j)], and
*L*₀ is a reference linker length, (L) is the DNA linker length, *k_{B}T* is the thermal energy.

Preferably, *L*₀, and *k_{B}T* are within the ranges as defined above.

The position and orientation data of the nucleosomes and/or the position and orientation data of the DNA linker arms on the nucleosomes used in step (i) may preferably be obtained from digital images. The images are not restricted and may be any digital image from any sample of chromatin as long as nucleosomes can be detected. Hence, the sample may be a sample of chromatin, chromosomes or parts thereof, inside cells or isolated therefrom. Preferably, the sample comprises, or consists of, eukaryotic tissues, eukaryotic cells or parts thereof. The origin of the cell is not restricted, and the cell may be any human or non-human cell. Preferably, the cells are human cells. The sample preparation is performed using techniques commonly used for the respective imaging technique used, and for example sample preparation may be performed as described in detail in the methods and examples section below.

Preferably, the position and orientation data of the nucleosomes and/or the position and orientation data of the DNA linker arms on the nucleosomes used in step (i) is obtained from a 3D digital image or also termed 3D digital volume. Preferably, the 3D digital image is a tomogram or a part thereof. Preferably, the tomogram is a 2x or 4x binned and/or denoised tomogram. More preferably, the tomogram is processed with any other algorithm that improves contrast or signal to noise ratio of the imaged nucleosomes and DNA linkers contained within.

The position and orientation data of the nucleosomes and/or the position and orientation data of the DNA linker arms on the nucleosomes used in step (i) may be obtained from images by any technique known in the art, such as for example by manually identifying the positions and/or orientations, by template matching techniques or by subtomogram averaging or by machine learning or artificial intelligence based image analysis algorithms.

Template matching is a technique in image processing used to find portions of an input image that matches a template image (S. Cruz-León, T. Majtner, P. C. Hoffmann, J. P. Kreysing, S. Kehl, M. W. Tuijtel, S. L. Schaefer, K. Geilßler, M. Beck, B. Turoňová, G. Hummer, High-confidence 3D template matching for cryo-electron tomography Nature Communications 15, 3 (2024).). Template Matching is commonly used for object detection, image recognition, and pattern recognition tasks. Template matching as also described in the methods and examples section in detail below may be used to obtain the position and orientation data of the nucleosomes in the cluster and/or the position and orientation data of the DNA linker arms on the nucleosomes.

Preferably, the position and orientation data of the nucleosomes in the cluster and/or the position and orientation data of the DNA linker arms on the nucleosomes used in step (i) is obtained by template matching.

Subtomogram averaging is a computational technique used in cryo-electron tomography to study the structure of macromolecular complexes, proteins, and other large biological assemblies at high resolution (Friedrich Förster; Subtomogram analysis: The sum of a tomogram's particles reveals molecular structure in situ, Journal of Structural Biology (6) 2022). Subtomogram averaging as also described in the methods and examples section below may also be used in combination with template matching to obtain the position and orientation data of the nucleosomes in the cluster and/or the position and orientation data of the DNA linker arms on the nucleosomes.

Machine learning or artificial intelligence based image analysis are computational techniques used to detect the position and orientation of macromolecular complexes in cryo electron tomograms (Irene de Teresa-Trueba, Sara K Goetz, Alexander Mattausch, Frosina Stojanovska, Christian E Zimmerli, Mauricio Toro-Nahuelpan, Dorothy WC Cheng, Fergus Tollervey, Constantin Pape, Martin Beck, Alba Diz-Muñoz, Anna Kreshuk, Julia Mahamid, Judith B Zaugg; Convolutional networks for supervised mining of molecular patterns within cellular context, Nature Methods (20) 2022). These methods may also be used in combination with template matching to obtain the position and orientation data of the nucleosomes in the cluster and/or the position and orientation data of the DNA linker arms on the nucleosomes.

More preferably, the position and orientation data of the nucleosomes in the cluster and/or the position and orientation data of the DNA linker arms on the nucleosomes used in step (i) is obtained from an image by template matching in combination with subtomogram averaging.

Preferably, the image is obtained by any imaging procedure such as, for example a microscopic technique. Preferably the imaging procedure is selected from electron microscopy, preferably electron tomography, preferably cryo-electron tomography, or light microscopy, preferably super-resolution light microscopy, or X-ray tomography.

A cluster of nucleosomes may comprise, or consist of, the entirety of nucleosomes present in an image as defined above or a part of the nucleosomes present in an image. Preferably, a cluster of nucleosomes comprises nucleosomes having a predefined distance to each other. Preferably the predefined distance is equal to or more than 1 nm to equal to or less than 1000 nm, most preferably equal to or more than 15 nm to equal to or less than 25 nm. More preferably, the nucleosomes have at least one of their DNA linker arms within said distance of at least one of the two DNA linker arms of the other nucleosome. Preferably, a cluster of nucleosomes comprises, or consists of, multiple nucleosomes in spatial proximity. Preferably, a cluster of nucleosomes comprises, or consists of, a subset of nucleosomes that is in close proximity and thus contains multiple candidate pairs of nucleosomes that may be connected by hypothetical linkages.

Clusters of nucleosomes may preferably be defined using the position and orientation data of nucleosomes which may be obtained from images as described above. By defining clusters of nucleosomes, the data comprised in an image may be divided into portions. Each portion, i.e. each cluster of nucleosomes may be processed separately according to the method of the invention. In other words, the method of the invention may be performed multiple times for a plurality of clusters of nucleosomes present an image. Accordingly, the advantage of defining clusters of nucleosomes is the reduction of computational cost.

A cluster of nucleosomes can be defined using established methods for distance-based clustering using position and orientation data of the nucleosomes obtained from an image and for example the distance based clustering implemented in cryoCAT (B. Turoňová, cryoCAT [software]. (available at https://doi.org/10.5281/zenodo.13916774)) may be used as described in detail in the methods and examples section below.

Preferably, a cluster of nucleosomes is determined by selecting a first nucleosome and identifying nucleosomes having a predefined distance to said nucleosome. The predefined distance is preferably equal to or more than 1 nm to equal to or less than 1000 nm, more preferably equal to or more than 2 nm to equal to or less than 500 nm, even more preferably equal to or more than 3 nm to equal to or less than 100 nm, even more preferably equal to or more than 4 nm to equal to or less than 50 nm, most preferably equal to or more than 15 nm to equal to or less than 25 nm to said nucleosome. Preferably the identified nucleosomes have at least one of their linker arms within said predefined distance of at least one of the two linker arms on the first nucleosome. Hence, preferably, the size of a cluster is equal to or more than 2 nm to equal to or less than 500 nm, even more preferably equal to or more than 3 nm to equal to or less than 100 nm, even more preferably equal to or more than 4 nm to equal to or less than 50 nm, most preferably equal to or more than 15 nm to equal to or less than 25 nm.

In the method for identifying the linkages between nucleosomes by DNA linkers in a cluster of nucleosomes, preferably the steps (i), and (ii) are conducted in that order.

The invention further provides a method for identifying the path of DNA along nucleosomes comprising, or consisting of, the steps of:
(i) identifying the linkages between nucleosomes by DNA linkers in one or more clusters of nucleosomes according to the method as described above,
(ii) identifying consecutively connected nucleosomes, thereby identifying the path of DNA along nucleosomes.

The identified path of DNA along nucleosomes together with these nucleosomes represents a patch of chromatin. The identified path of DNA along nucleosomes can also be termed as a path of DNA through nucleosomes according to the "beads on a string model", wherein the linker DNA is seen as the string and the stretches of DNA between the DNA linkers are wound around the nucleosomes. Preferably, further the sequence of the DNA linkers between the consecutively connected nucleosomes is identified. The sequence starts at the 1^{st} nucleosome of the consecutively connected nucleosomes and extends to the last nucleosome of the consecutively connected nucleosomes.

The more consecutively connected nucleosomes are identified, the longer the identified path of DNA along nucleosomes. Preferably, the consecutively connected nucleosomes are connected by at least two, preferably at least three, more preferably at least five, most preferably at least seven consecutive DNA linkers. Accordingly, there is no upper limit for the number of consecutively connected nucleosomes and thus the number of consecutive DNA linkers, however usually not more than 100 consecutive DNA linkers between consecutively connected nucleosomes are identified.

Preferably, for identifying the sequence of the DNA linkers between consecutively connected nucleosomes, consecutively connected nucleosomes are identified.

Preferably, the identified path of DNA along nucleosomes may be present in one cluster of nucleosomes or may extend over a plurality of clusters of nucleosomes. As described above a cluster of nucleosomes may comprise, or consist of, the entirety of nucleosomes present in an image as defined above or a part of the nucleosomes present in an image. Hence, preferably the clusters of nucleosomes wherein linkages between nucleosomes by DNA linkers are identified may be overlapping.

An exemplary path of DNA along nucleosomes is shown in Fig. 2d

The invention further provides a method for determining the nucleosome distance pattern of a patch of chromatin comprising, or consisting of, the steps of:
(i) identifying the path of DNA along nucleosomes,
(ii) calculating a plurality of the lengths of the DNA of the pairs of nucleosomes of the path, wherein the length of the DNA of a pair of nucleosomes comprises, or consists of, the DNA linker length (L) and the length of the DNA wound up on one nucleosome,
wherein the plurality of lengths of DNA of the pairs of nucleosomes of the path defines the nucleosome distance pattern of the patch of chromatin.

In one preferred embodiment, the path of DNA along nucleosomes of step (i) may be identified directly in digital images, preferably as defined above. Accordingly, preferably the identified path of DNA along nucleosomes of step (i) is obtained directly from digital images. The images are not restricted and may be any digital image from any sample of chromatin as long as the path of DNA can be detected. The sample may be a sample as defined above. Preferably, step (i) further comprises obtaining the path of DNA and/or the position and orientation data of the path of DNA, preferably including the nucleosome data from digital images. The path of DNA and/or the position and orientation thereof may be obtained from images by any technique known in the art, such as for example by manually identifying the positions and/or orientations, by template matching techniques or by subtomogram averaging or by machine learning or artificial intelligence based image analysis algorithms as defined above. Preferably, the path of DNA and/or the position and orientation thereof is obtained by template matching, more preferably by template matching in combination with sub tomogram averaging. Preferably, the image is obtained by any imaging procedure as defined above.

In a further more preferred embodiment, the path of DNA along nucleosomes of step (i) is identified according to the method for identifying the path of DNA along nucleosomes as defined above.

A nucleosome distance pattern comprises information about the length of the DNA linkers within a patch of chromatin, i.e. multiple consecutive nucleosomes. A pattern consists of multiple segments, each comprising a nucleosome N, a DNA linker and a nucleosome N+1. Thereby, the distance between each nucleosomes pair (N, N+1) within a segment is given by the sum the lengths of the DNA linker connecting from nucleosome N to nucleosome N+1 (variable number) and the DNA wrapped around one histone octamer (147 bp).

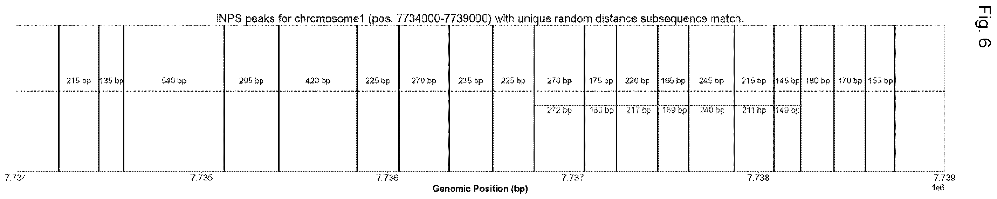

Accordingly, the DNA of a pair of nucleosomes comprises, preferably consists of, the DNA between the pair of nucleosomes, i.e. the DNA linker length (L) and the DNA wound up on one nucleosome. The length of the DNA of a pair of nucleosomes is calculated for a plurality of the pairs of nucleosomes of the path. Preferably, the length of the DNA of a pair of nucleosomes is calculated for all of the pairs of nucleosomes of the path. Each of the DNA stretches of a pair of nucleosomes of the path has a unique length. Hence, the nucleosome distance pattern of a patch of chromatin thus comprises, preferably consists of a plurality of these unique lengths. An exemplary nucleosome distance pattern of a patch of chromatin is shown in Fig. 6.

Preferably, the DNA linker length (L) is determined as described above. The length of the DNA wound up on one nucleosome is usually the length of 147 base pairs. The standard rise per base pair in B-DNA is 0.34 nm. Accordingly, in B-DNA the length of the DNA wound up on one nucleosome is usually 50 nm.

The invention further provides a method for identifying the genomic position of a patch of chromatin comprising, or consisting of, the steps of:
(i) determining the nucleosome distance pattern of a patch of chromatin according to the method as described above,
(ii) determining matches between said nucleosome distance pattern of the patch of chromatin (i) and reference DNA length patterns of chromatin obtained from databases, thereby identifying the genomic position.

Preferably, the patch of chromatin and accordingly the nucleosome distance pattern of the patch of chromatin comprises at least two, preferably at least three, more preferably at least five, most preferably at least seven consecutively connected nucleosomes. There is no upper limit for the number of consecutively connected nucleosomes, however the nucleosome distance pattern of a patch of chromatin comprises usually not more than 100 consecutively connected nucleosomes.

The databases from which the reference nucleosome distance patterns of chromatin are obtained are not restricted as long as they provide the nucleosome positioning genomic data. Genome-wide nucleosome positioning maps are now available for many model organisms and human cells such as for example the NucMap database (Zhao et al. Nucleic Acids Res 2019).

For determining matches between nucleosome distance patterns, preferably the nucleosome distance patterns have to be compared. Hence, for determining the matches between the nucleosome distance pattern of a patch of chromatin (i) and a reference nucleosome distance pattern of chromatin, preferably the patterns have to be compared. The nucleosome distance pattern of a patch of chromatin (i) may be compared to one or more reference nucleosome distance pattern obtained from different databases. Preferably, the nucleosome distance pattern of a patch of chromatin (i) is compared to one reference nucleosome distance pattern.

A nucleosome distance pattern may disclose the lengths of the DNA between the nucleosomes in nm or in base pairs. Hence, for comparing length patterns, it may be that one nucleosome distance pattern has to be converted.

Usually, nucleosome positioning genomic data disclose the lengths of the DNA between the nucleosomes in base pairs. The nucleosome distance pattern of a patch of chromatin according to the method as described above provides the lengths of the DNA between the nucleosomes in nm. Accordingly, preferably the length pattern of the patch of chromatin (i) and/or the reference patterns of chromatin are converted to be in a mutually comparable format.

The nucleosome positioning genomic data may also disclose the nucleosomes positions as nucleosome density function along the DNA. In this case, preferably the peaks representing nucleosome positions have to be extracted and their center position is equivalent to the position of nucleosomes of the DNA. Accordingly, preferably the linker length between two adjacent nucleosomes is the distance of the same two nucleosomes subtracted by the length of the DNA wrapped around one nucleosome, i.e. 147 bp or 50 nm.

Preferably, the length pattern of the patch of chromatin (i) is converted into a length pattern in base pairs by converting the lengths of the DNA between the pairs of nucleosomes comprising the DNA linker length and the length of the DNA wound up on one nucleosome by using the standard rise per base pair in B-DNA, preferably using a rise per base pair of 0.34 nm. Hence, preferably the DNA linker length between the nucleosomes determined as described above is converted using n_{bp}≈L/(0.34 nm) each length of the DNA between the pairs of nucleosomes

Preferably, the nucleosome positioning genomic data obtained from a database is converted to a reference length pattern of chromatin by:
(i) defining that the center position of a nucleosome peak obtained from the database is the center of a nucleosome,
(ii) thereby obtaining the lengths of the DNA between the nucleosome centers from the nucleosome positing genomic data, thereby receiving the reference length pattern of chromatin.

Preferably, the matches between said nucleosome distance pattern of the patch of chromatin of (i) and reference nucleosome distance patterns of chromatin obtained from databases are determined for both directions of the length pattern of the patch of chromatin of (i). The advantage thereof is that the credibility of the match is improved and thus allows for a more unambiguous assignment of a patch of chromatin to a genomic position. This may be particularly useful in case the position and orientation data of the nucleosomes and/or the position and orientation data of the DNA linker arms on the nucleosomes is obtained from low resolution images.

Preferably, a match is defined as present if the nucleosome distance pattern of the patch of chromatin (i) and reference nucleosome distance patterns of chromatin obtained from databases are similar. The length patterns are similar and accordingly a match is defined as present if the length patterns are identical to at least 50%. Preferably, a match is defined as present if the length patterns are identical to at least 80%, more preferably to at least 90%, most preferably to at least 95%. A match is defined as present if the length patterns are identical to 100%.

Preferably, a preset tolerance is permitted, when determining the matches between the nucleosome distance pattern of (i) and the reference nucleosome distance patterns. Preferably, a match is defined as present if the nucleosome distance pattern of the patch of chromatin (i) and reference nucleosome distance patterns of chromatin obtained from databases are identical within a preset tolerance.

The preset tolerance may be adjusted depending on the quality of the position and orientation data of the nucleosomes and/or the position and orientation data of the DNA linker arms on the nucleosomes and on the number of nucleosomes present in the length pattern of the patch of chromatin. A higher quality of the data and a higher number of nucleosomes present in the nucleosome distance pattern of the patch of chromatin allows for a higher preset tolerance. A lower tolerance is used in case quality of the data is low and the length pattern comprises a low number of nucleosomes.

Preferably, the preset tolerance is equal to or not more than ± 20 base pairs or equal to or not more than ± 7 nm for each distance between the nucleosome pairs of the nucleosome distance pattern of (i), preferably the preset tolerance is between ± 1 base pair and ± 18 base pairs or between ± 0.3 nm and 6.1 nm, preferably between ± 3 base pairs and ± 10 base pairs or between ± 1.0 nm and 3.4 nm, most preferably the preset tolerance is equal to or not more than ± 6 base pairs or equal to or not more than ± 2 nm.

The invention further provides a method for determining the nucleotide sequence of DNA comprising the steps of:
(i) identifying the genomic position of a patch of chromatin according to the method as described above, thereby obtaining the nucleotide sequence of said genomic position from the database.

The nucleotide sequence of a genomic position is present in the database used for identifying the genomic position of a patch of chromatin, preferably in the nucleosome positioning genomic data. Hence, by identifying the genomic position of a patch of chromatin the nucleotide sequence of a patch of chromatin is obtained automatically. The invention thus provides an entirely new method for determining the nucleotide sequence of DNA.

In the inventive methods, preferably the steps (i) and (ii) if present are conducted in that order.

Usually, the inventive methods as described above are implemented into a software and are performed using computers. Hence, a plurality of the steps of the inventive methods may be, preferably are, performed *in silico.* Furthermore, preferably, a plurality of the steps of the inventive methods are performed automatically. Accordingly, the inventive methods may be, preferably are, computer-implemented methods. Data relating to the position and spatial orientation of the nucleosomes in the cluster as defined above are provided to the computer and processed as described above.

Furthermore, the invention provides a computer program comprising instructions, which, when the program is executed by a data processing device, cause said device to carry out the steps of methods as described above.

Furthermore, the invention provides a data processing system comprising means for carrying out the steps of the methods as described above. Such data processing system may be implemented as a computer, a personal computer, a tablet, a workstation or a smartphone.

Furthermore, the invention provides a computer-readable medium comprising instructions which, when executed by data processing system such as for example by a computer, cause the data processing system to carry out the steps of the methods as described above.

The method for identifying the path of DNA along nucleosomes, the method for determining the nucleosome distance pattern of a patch of chromatin, the method for identifying the genomic position of a patch of chromatin and the method for determining the nucleotide sequence of DNA may use, or preferably use, the steps of the method for identifying the linkages between nucleosomes by DNA linkers in a cluster of nucleosomes. Accordingly, all embodiments and preferred aspects described for the method for identifying the linkages between nucleosomes by DNA linkers in a cluster of nucleosomes are *mutatis mutandis* applicable to the method for identifying the path of DNA along nucleosomes, the method for determining the nucleosome distance pattern of a patch of chromatin, the method for identifying the genomic position of a patch of chromatin and the method for determining the nucleotide sequence of DNA.

Similarly, all embodiments and preferred aspects described for the method for identifying the path of DNA along nucleosomes are *mutatis mutandis* applicable to the method for determining the nucleosome distance pattern of a patch of chromatin, the method for identifying the genomic position of a patch of chromatin and the method for determining the nucleotide sequence of DNA.

Similarly, all embodiments and preferred aspects described for the method for determining the nucleosome distance pattern of a patch of chromatin are *mutatis mutandis* applicable to the method for identifying the genomic position of a patch of chromatin and the method for determining the nucleotide sequence of DNA.

Similarly, all embodiments and preferred aspects described for the method for identifying the genomic position of a patch of chromatin are *mutatis mutandis* applicable to the method for determining the nucleotide sequence of DNA.

The present invention is further illustrated by the below methods and examples which refer to the following figures:

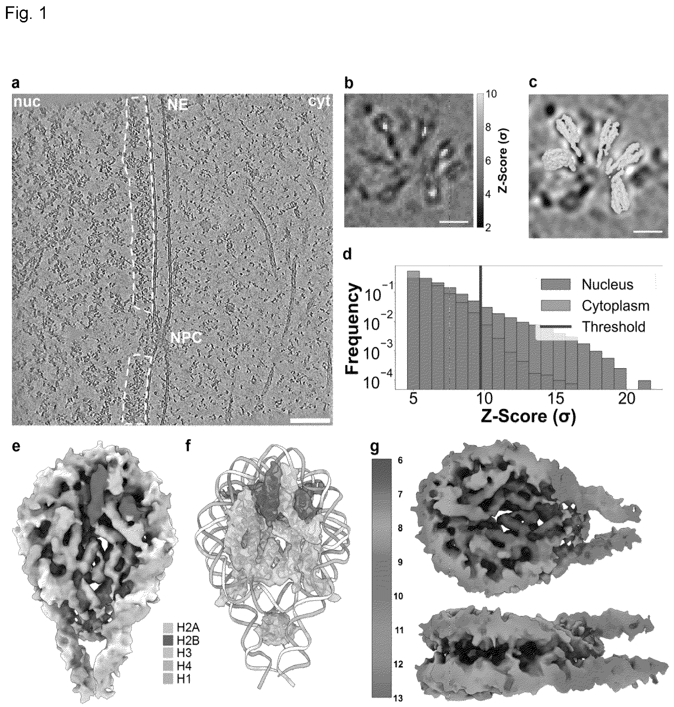

**Figure 1****: In situ structural analysis identifies nucleosomes inside of cells and resolves their secondary structure.**
   a Slice through a cryoCARE-denoised (27) tomogram acquired at the nuclear periphery of resting T cells. Chromatin-free space is indicated with an arrow, a dense layer of chromatin underlying the nuclear envelope is framed white. **b** A local feature of multiple nucleosomes superimposed with the respective cross-correlation function obtained by template matching. **c** The same local feature but with the identified nucleosomes shown as isosurfaces. **d** Histogram of Z-scores for the cytosolic and nucleoplasmic region for exemplary tomogram. **e,f** STA map of the human chromatosome, grey-coded by DNA strands (outside) and histone proteins H2A, H2B, H3, H4 and H1 (insides shades of grey). The atomic model of the canonical chromatosome (PDB: 7dbp (26)) was fitted into our map. Scale bars 100 nm in a and 10 nm in b,c.
**Figure 2****: Cryo-ET and physics-based model allow kbp-long chromatin to be visualized.**
   **a-c** A physics-based model of nucleosome pairs is used to add DNA linkers (intermediate grey) to in situ nucleosome DNA (white) (**a**). All four distinct possibilities to connect two nucleosomes with two linkers each are scored, resulting in connectivity matrix for all nucleosomes within a given cluster (see Methods and Fig. 3 for an example) (b). The connectivity matrix from b is represented as graph, with nucleosomes as nodes connections as edges (c). Nodes are projected on the x-y plane and colored according to the z-coordinate. Edge thickness and color are proportional to the connection probability. Assigned linkers are shown as solid arrows between nodes. d Visualization of an exemplifying tomogram (corresponding to Fig 1a) showing the densely interconnected chromatin structure within the nucleus and identified linkers. The nuclear envelope membrane was segmented with MemBrain-seg (31).The zoom-in is the 3D representation of 60 traced nucleosomes in b-c.
**Figure 3****: Visualization of the linker assignment. a** Scheme for determining the parameters to for the probability of DNA linkers. The probability of a DNA potential linker (dashed line) to exist is calculated from the bending angle, *θ*, and the arc length L. For each pair of nucleosomes *i,j,* and each combination Γ of connections between the DNA linker arms, the length(L)is determined as the arc length measure from the center of bp 1 or bp 147 in the nucleosome (points aᵢ, and aⱼ). The bending angle is determined as *θ* = $\frac{{θ}_{i} + {θ}_{j}}{2}$. **b** Set of possible connections Γ between a pair of nucleosomes (*i, j*) shown in shades of grey, whereby the DNA linker with the lowest bending energy is shown as thick intermediate grey connection. **c** Algorithm for assigning linker connections. The columns show the graph of the nucleosomes (left), the probability matrix P (middle) obtained as described in Methods, and the visualization of the nucleosome position and the predicted linkers (right). The linkers are assigned iteratively, starting with the configurations with the highest probability *Pₘₐₓ.* In the example shown the linker (3↔6) is created in the first iteration. For the next iteration, we set *P*_{36*k*} = *P*_{63*k*} = 0, ∀ *k* ∈ [0,1,2,3], and now *Pₘₐₓ* creates the connection (2↔9). The process continues until no further connections are possible with a threshold *Pᵢⱼₖ* > 0.1. Note that the indices of the particles is arbitrary.

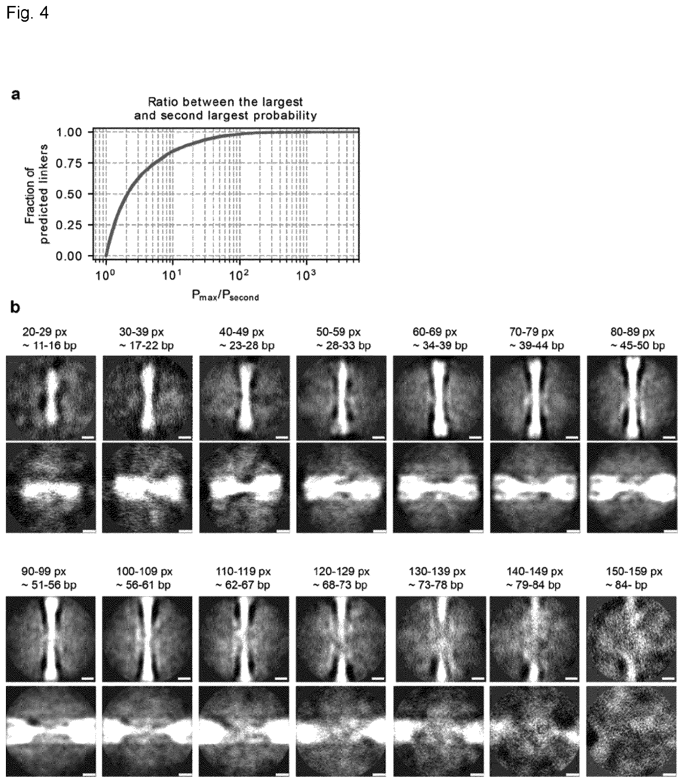

**Figure 4** **Predicted DNA linker validation through Pmax/Psecond and STA. a** Cumulative distribution of the ratio between the highest probability Pₘₐₓ and the second highest probability P_{second}, for all the assigned DNA linkers (see Methods). This ratio quantifies competing linkage possibilities, with larger values indicating higher confidence in the linkage assignment. **b** The DNA linkers were grouped by predicted length and subjected to separate STA refinements in Relion 3.1(49). YZ and XZ slices through each linker DNA average are shown with the predicted length matching well to increasing length of the densities.

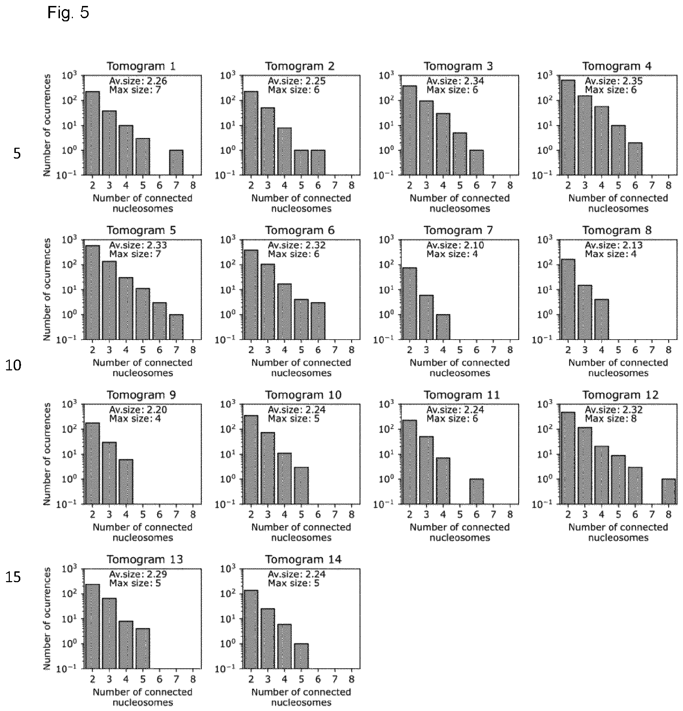

**Figure 5****: Histograms of the number of connected nucleosomes per tomogram.** Distribution of the number of connected nucleosomes per tomogram after the assignment of linker connections. The inset text shows the maximum number of connected nucleosomes per tomogram, as well as the average size.
**Figure 6****:** Nucleosome distance pattern for small section of chromosome 1 from T cell dataset (hsaNuc0000225) with a unique match to a random search subsequence of distances. Shown are nucleosome positions (vertical lines) in a segment of chromosome 1 and their pair-wise separations. An exemplifying search sequence (2nd line) could be uniquely matched across the whole human genome annotated by this dataset to part of the section shown for chromosome 1 (with an error tolerance of 5 bp).

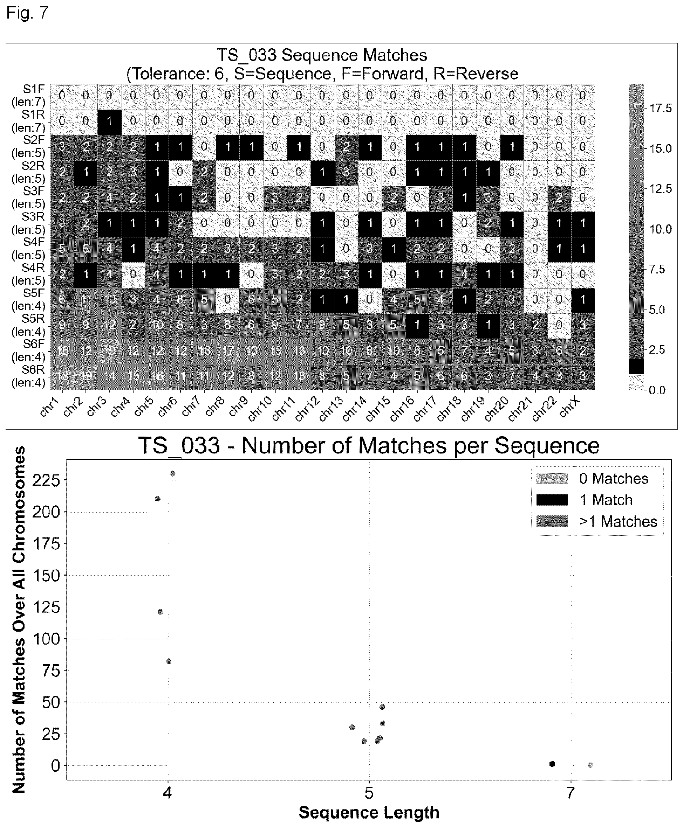

**Figure 7****: Nucleosome distance pattern experimentally observed in an exemplifying tilt series matched to genomic data leads to an unambiguous identification of genome position.** Top: heat map showing the number of matches per chromosome for all nucleosome patches extracted from an exemplifying tomogram. For a pattern that connects 7 nucleosomes, a single match on Chromosome 3 is obtained. All other patterns that contain 4 or 5 nucleosomes are not unambiguous but also contain matches on Chromosome 3 (len: length of pattern; F: forward; R: reverse). Bottom: Dependencies of the number of matches on the length of the pattern (Red: forward; Green: reverse).

### Examples

### Example 1

### Materials and Methods

### Preparation of primary CD4+ T cells

Detailed CD4+ T cells purification method used is described in Lucic et al., 2019. Briefly, cells were obtained by using RosetteSep Human CD4+T cells enrichment cocktail (STEMCELL technologies) and isolated by Ficoll gradient centrifugation. Following the lysis of red blood cells, purified T cells were cultured in RPMI-1640 10% FBS and penicillin/streptomycin, in the presence of IL2 10 ng/µL and incubated at 37°C in a 5% CO2 atmosphere. Cells were then transferred to 20mM Hepes pH 7.9 RPMI-1640 at a concentration of 4 × 10⁶ cells for further use.

### Cell vitrification and cryo-FIB milling

3.5 µL of cell suspension of resting primary human CD4+ T cells was applied on glow discharged 200-mM mesh EM gold grids (Quantifoil Micro Tool GmbH) coated with R 2/2 holey SiO2 films and plunge frozen in liquid ethane -183°C using a Leica EM GP2 grid plunger (Leica Microsystems) with the blotting chamber at 37°C. The grids were blotted from the back (cell-free) side for 4-5 seconds with a Whatman filter paper, Grade 1 before plunge freezing.

The cells were then cryo-FIB milled using an Aquilos 2 microscope (Thermo Fisher Scientific) in a similar fashion to a previously described workflow (41). In brief, samples were coated with an organometallic platinum layer for 10 sec and additionally sputter coated with platinum at 1 kV and 10 mA current for 10 sec. The lamella milling was performed with AutoTEM (version 2.4.2) (Thermo Fisher Scientific) in a stepwise manner with a 30kV ion beam while reducing the current from 1000 pA to 50 pA. Final polishing was performed with 30 pA current with a lamellae target thickness of 200 nm.

### Cryo-ET data acquisition

Cryo-ET data for resting T-cells were collected at 300 kV on a Titan Krios G4 microscope (Thermo Fisher Scientific) equipped with a E-CFEG, Falcon 4 direct electron detector (Thermo Fisher Scientific) operated in counting mode and Selectris X imaging filter (Thermo Fisher Scientific). Montaged grid overviews were acquired for each grid with a 205 nm pixel size. Individual lamellae montages were taken with 30 nm pixel size. Tilt series were acquired using SerialEM (version 4.0.20) (42) in low dose mode as 4K × 4K movies of 10 frames each and on-the-fly motion-corrected in SerialEM. The magnification for projection images of 64000x corresponded to a nominal pixel size of 1.971 Å. Tilt series acquisition was started from the lamella pretilt of ± 8° and a dose-symmetric acquisition scheme (43) with 2° increments grouped by 2 was applied, resulting in 61 projections per tilt series with a constant exposure time and targeted total dose of ~135 e- per Å2. The energy slit width was set to 10 eV and the nominal defocus was varied between - 1.75 to -4.25 µm. Dose rate on the detector was targeted to be ~5 e- /px/sec.

### Tomogram reconstruction

Dose exposure correction for the motion corrected tilt series was performed as previously described (44) using a Matlab implementation that was adapted for tomographic tilt series (45). Poor quality projection images were removed after visual inspection. The dose-filtered tilt series were then aligned with patch-tracking in AreTomo (v2.0) (46) and reconstructed as back-projected tomograms with SIRT-like filtering of 15 iterations at a binned pixel size of 7.884 Å (bin4) in IMOD (version 4.11.5) (47). The 14 tomograms containing a nuclear envelope were selected for further processing. These tomograms were then denoised with the cryoCARE software package (27) for improved visualization.

For the same tomograms, reconstruction with 3D-CTF correction was also performed using novaCTF (23) with phase-flip correction, astigmatism correction and 15 nm slab. Tomograms were binned 2x and 4x using Fourier3D (48). For compatibility with Relion 3.1 (49) and M (50) these 14 tilt series were reprocessed in Warp (51) with the alignment obtained from AreTomo (46).

### Particle coordinates and orientations by nucleosome template matching, including initial template generation

To obtain initial particle coordinates and orientations, the bin2 (3.942 Å/px) 3D-CTF corrected tomograms were template matched with an existing nucleosome structure (EMD-3947) (lowpass filtered to 13 Å) and 5 degree global angular sampling in GAPSTOPTM (24, 25). The resulting constrained cross correlation (CCC) volumes were used to extract positions and coordinates of peaks with a CCC score 4.5 sigmas above the mean CCC score value. The candidate particles determined through template matching were extracted as subtomograms in Warp (v1.09) (51) at bin2. Subsequent junk classification and refinement in Relion (v3.1) (49) lead to an initial STA nucleosome structure that was then used as an improved search template for further template matching.

To improve template matching performance, we used the cryoTIGER computational workflow (22) to interpolate between tilt stacks using the FILM algorithm (52), as detailed in (22). The aligned bin2 tilt stacks were used as inputs for cryoTIGER where one tilt image was interpolated in between two real tilt images for each stack. The combined stacks containing real and interpolated tilt images were used for novaCTF (23) reconstruction using the same parameters as above. The resulting tomograms were template matched with the generated initial STA nucleosome structure without DNA linker arms (lowpass filtered to 13 Å) and 5 degree global angular sampling in GAPSTOPTM (24, 25). Since all tomograms contained both cytoplasm and nucleoplasm the two regions were manually segmented in IMOD (47) for each tomogram. The constrained cross-correlation (CCC) peaks in both regions of the tomogram could thereby be compared and a peak extraction threshold for nucleoplasmic peaks could be set per tomogram. This threshold corresponded to the 99th percentile of CCC values for cytoplasmic peaks in each tomogram (Fig 1d), allowing for extraction of a high-confidence nucleosome particle list. This particle list was further cleaned by excluding clashing particles with a nucleosome shape mask around each peak in cryoCAT (30).

### Nucleosome subtomogram averaging

The 33,560 particles determined through template matching were extracted as bin1 (1.971 Å/px) subtomograms in Warp (51) (no interpolation between tilt stacks) and subjected to subtomogram averaging and alignment in Relion 3.1 (49). Multiple attempts at classifying out junk particles were made, all resulting in no significant percentage of particles being classified as junk. The particle set was then imported into M (50) to perform multi-particle refinement of the tilt-series and the nucleosome. Geometric and CTF parameters were refined in a sequential manner. Afterwards, new M-corrected bin1 (1.971 Å/px) subtomograms were again refined in Relion 3.1. This resulted in a chromatosome structure resolved to 7.7 Å with C1 symmetry and a 7.3 Å chromatosome structure with C2 symmetry imposed (at FSC 0.143 cutoff). Locally the chromatosome with C2 symmetry imposed was resolved up to 6.4 Å. The particles refined with C1 symmetry were furthermore classified based on potential stacking neighbors and around the H1 linker histone binding site.

### Physics-based model and algorithm for determining DNA linkers between nucleosomes

We have developed a physics-based algorithm to infer linker connections between nucleosomes in tomograms. The algorithm utilizes the positions and orientations of nucleosomes obtained after M-refinement to compute the energy required for linker formation, describing the DNA with the wormlike chain (WLC) model. Based on these energy calculations, probabilities are determined for all possible nucleosome pairings and their respective linker configurations. Linkers are then assigned iteratively, beginning with the configurations of the highest probability. The subsequent sections describe the theory, implementation details, and algorithm validation.

**Theory:** Nucleosomes are connected by double-stranded (ds)DNA linkers. Single-molecule elasticity measurements have shown that the entropic elasticity of dsDNA in aqueous solutions is accurately described by the WLC model with a fixed contour length (55-57). Deviations of the chain from its straight configuration result in a bending energy *U_{bend}* (58). To second order, we approximate the DNA bending energy as (59). ${U}_{bend} \left(θ/2\right) = \frac{2 {l}_{p}}{L} {\left(\frac{θ}{2}\right)}^{2} {k}_{B} T$ where *lₚ* ≈ 50 nm is the persistence length of DNA (60) at physiological conditions, *L* its length, *θ* its bending angle, *k_{B}* the Boltzmann constant, and *T* the temperature. We use equation (2) to estimate the energy required to bend a segment of DNA into a linker between the selected arms of a pair of nucleosomes.

**Implementation:** Starting from the nucleosomes' coordinates and orientations, we want to determine the most likely trajectory of the DNA that connects two nucleosomes. From the map obtained from STA (Fig 1e-g), the approximate direction of the two linker arms in the nucleosome is determined (See Fig. 3a). We denote the linkers of the nucleosome i as: Linker1*ᵢ*, and Linker2*ᵢ*. There are 4 possible ways Γ to connect a pair of nucleosomes *(i, j*), that is: Γ ∈ [(Linker1*ᵢ*: Linker), (Linker1*ᵢ*: Linker2*ⱼ*), (Linker2*ᵢ*: Linker1*ⱼ*), (Linker2*ᵢ*: Linker2*ⱼ*)]. For each pair of nucleosomes *i,j,* and each combination of Γ, we determine the probability of this connection as: ${P}_{ij} \left(L, θ, Γ\right) = \left({e}^{\frac{- L \left(Γ\right)}{{L}_{0}}}\right) \left({e}^{\frac{- Δ {U}_{bend} \left(θ\left(Γ\right)\right)}{{k}_{B} T}}\right)$

Note that the length L and the angle *θ*, depend on the case Γ (see Video S1). In Equation (5), we penalize long linker lengths as our uncertainty increases. We set *L*₀ = 15 nm as an average value of the expected linker length since DNA linkers range from ~20-90 bp and vary among different species, tissues, and even fluctuate within a single cellular genome (61).

To determine *Pᵢⱼ*, we computed *Lᵢⱼ*(Γ) and *θᵢⱼ*(Γ) for a given connection Γ from the tangent vectors ***T**ᵢ, **T**ⱼ* and the connecting vector ***D**ᵢⱼ* (See Fig. 3a).

**Calculation of the probability matrix** P: To identify the most likely nucleosome connections, we computed the probability matrix *P,* which estimates *Pᵢⱼ*(*L, θ*, Γ) for all pairwise combinations and cases Γ. To reduce the computational cost, the particle list was pre-processed using cryoCAT (52). Using the previously described chain tracing algorithm (62), nucleosomes that are spatially close to each other were grouped into chains. For the chain tracing, nucleosome coordinates were shifted to the position of bp 1 and bp 147 (points ai and aj in Fig. 3a), generating two coordinate sets per nucleosome. Tracing decisions were based solely on the distance between ai and aj, with a maximum distance of 20 nm. For each traced cluster *M*, a pairwise probability matrix *P_{M}* ∈ R^{*N*×*N*×4} was calculated (See Fig. 2b and Fig. 3c), where |M|=N is the number of particles in the traced cluster, and the third dimension of the matrix corresponds to the four possible configurations, |Γ|=4.

**Assignment of linker connections:** From the matrix P, we iteratively established the connectivity between particles. First, we defined: $C = \left\{{C}_{1},{C}_{2},…,{C}_{N}\right\} ,$ where *C*ᵢ represents the set of nucleosomes that are connected to nucleosome *i* and the configuration Γ of the connection. Each particle can have at most one connection at linker 1 and one at linker 2.

For the iterative assignment, we identified the position *(i,j,k)* with the highest probability *Pᵢⱼₖ = max(P)* and checked if the connection (i↔j) was possible based on the topological constraints mentioned above. If the connection is valid, we added (i↔j) to *Cᵢ,* and (j↔i) to *Cⱼ.*

Next, we set *P*_{*ij*0} *= P*_{*ij*1} = *P*_{*ij*2} = *P*_{*ij*3} = 0 and *P*_{*ji*0} *= P*_{*ji*1} *= P*_{*ji*2} *- P*_{*jj*3} = 0, thereby removing the pair of nucleosomes from future iterations. This process continued until no more connections were possible, imposing a a probability threshold, *Pᵢⱼₖ* > 0.1. At each iteration, it was also checked that there are no closed loops.

The connectivity structure C can be represented as a graph *G(V.E)* where, V are the nucleosomes (nodes) and E are the established connections (edges). See Fig. 2c and FIG. 3c.

### Validation of the predicted linkers

Using the final connectivity graph G, wea estimated the coordinates and orientation of the linker centers. A particle was placed at the geometric center between the predicted connected arms, and the orientation of the linkers was assigned to align with the vector connecting the corresponding linker arms (See Fig. 3a).

**Subtomogram averaging of the predicted linkers:** We performed STA in Relion3.1 (49) on the 7163 predicted linkers using the positions and orientations derived from the algorithm (see above) with subtomograms being extracted with Warp (51), as before no interpolation was used for the STA. The average of the extracted subtomograms was used as the initial reference to avoid biasing the refinement. As a further test, we grouped the linkers based on their predicted length (Fig 4c) and performed STA on each group (see Fig 4), resulting in cylindrical shapes with lengths consistent with the predictions.

**P_max /P_second of the predicted linkers:** To evaluate the certainty of linker assignments in the connectivity analysis, we recorded the highest probability P_max and the second highest probability P_second, at each iteration of the algorithm where a linker was assigned. Specifically, P_max= max{P_ijk | ∀ i,j,k} =P_(i_0 j_0 k_0 ) represents the highest probability for the assigned connection between a pair of particles i and j, reflecting the most confident linkage based on the computed probabilities. On the other hand, P_second= max{P_(i_0 jk) |∀j,k≠j_0,k_0} is the second-highest probability value from the same row in the probability matrix, serving as a measure of competing linkage possibilities. A large ratio P_max \/P_second indicates a more confident linkage assignment (see Fig 5).

### In sillico determination of a number of segments in a nucleosome distance pattern that is sufficient for genomic matching

Nucleosome peaks with respect to their genomic position from a T-cell lymphoma cell line were obtained from a published dataset (iNPS peaks from hsaNuc0000225 in the NucMap database (Zhao et al. Nucleic Acids Res 2019) and the distances between the centers of the peaks along the DNA sequence were calculated **(****Fig. 6****).** Exemplifying randomly chosen subsequence of different lengths were matched against the entire genome-wide distance set with an error tolerance of at most 5 bp for each distance. To check for consistency, the procedure was repeated ten times for different randomly chosen subsequences. and observed each time that only one unique match could be found in the entire genomic sequence.

### Matching of nucleosome distance patterns observed in cryo electron tomograms to genomic data of nucleosome positioning

iNPS peaks were obtained from the dataset hsaNuc0000225 (wildtype T cell). The .bed peaks file contains the columns "chrom", "start", "end", "name". For each row the center of the nucleosome was determined by adding the genomic position of "start" and "end" and then dividing by two. The distances between these nucleosome centers were determined, resulting in a data structure containing the sequence of nucleosome distances for each chromosome and the respective genomic position. The contour length of a DNA linker in the tomograms was translated to its number *n_{bp}* of basepairs by using the standard rise per basepair in B-DNA, *n_{bp}* ≈ L/(0.33 nm). The canonical 147 bp wound up on the nucleosome was added, resulting a nucleosome distance along the genome of *n_{bp}* + 147 ± 6 bp. To account for imprecisions in the dataset and our search distance sequence an error tolerance of 6 bp was considered during matching. The algorithm matches the search sequence of a given nucleosome distances pattern observed between connected nucleosomes in the cryo electron tomograms to the database of nucleosome distances for each chromosome. To account for the directionality of the search sequence (left-to-right or right-to left) that remains undetermined, both directionalities are matched.

### Results

### Acquisition and initial processing of imaging data

To study multi-nucleosome arrangements in their native state, resting T cells from human donors were vitrified and subjected to specimen thinning by focused ion beam (FIB) milling as described above. 14 tilt series were further processed with a projection interpolation algorithm, cryoTIGER (Y. Li, H. Zhang, X. Li, W. Wu, P. Zhu, Cryo-ET study from in vitro to in vivo revealed a general folding mode of chromatin with two-start helical architecture. Cell Rep 42, 113134 (2023)), to improve angular sampling reconstructed in novaCTF (B. Turoňová, F. K. M. Schur, W. Wan, J. A. G. Briggs, Efficient 3D-CTF correction for cryo-electron tomography using NovaCTF improves subtomogram averaging resolution to 3.4Å. J Struct Biol 199, 187-195 (2017)) and template matched using GAPSTOPTM (S. Cruz-León, T. Majtner, P. C. Hoffmann, J. P. Kreysing, S. Kehl, M. W. Tuijtel, S. L. Schaefer, K. Geißler, M. Beck, B. Turoňová, G. Hummer, High-confidence 3D template matching for cryo-electron tomography Nature Communications 15, 3 (2024). B. Turoňová, GAPStop(TM) - GPU Accelerated Python-base Stopgap for Template Matching [software]. (available at https://doi.org/10.5281/zenodo.10822455).) with a nucleosome structure (Fig 1 a,b,c) (see Methods and Fig S1 for detail). The resulting cross-correlation peaks (Fig 1b) visually coincided with the particles that resembled nucleosomes (Fig 1c). These were considerably more frequent in the nucleoplasm as compared to the cytosol (Fig S2), in line with the expected localization of chromatin. We therefore set the threshold for peak extraction at the 99th percentile of cytoplasmic peaks (Fig 1d, see Methods for details). An about 50 nm thick layer was observed densely packed with nucleosomes underneath the inner nuclear membrane (Fig 1a), which is consistent with recent findings on lamin-nucleosome interactions in Murine Embryonic Fibroblasts (B. Wang, R. Kronenberg-Tenga, V. Rosti, E. Di Patrizio Soldateschi, Q. Luo, L. Pinet, M. Eibauer, R. Boujemaa-Paterski, B. Schuler, C. Lanzuolo, O. Medalia, The molecular basis of lamin-specific chromatin interactions bioRxiv 0 (2024)).

### Validation of nucleosome positions using subtomogram averaging (STA)

STA (performed with non-interpolated data) confirmed that the extracted peaks primarily account for nucleosomes. An in situ structure of the chromatosome from resting T cells locally resolved to 6.4 Å (7.3 Å overall) with imposed C2 symmetry was obtained (Fig 1e). It closely resembles previous *in vitro* structures (S. Wang, V. K. Vogirala, A. Soman, N. V. Berezhnoy, Z. B. Liu, A. S. W. Wong, N. Korolev, C. J. Su, S. Sandin, L. Nordenskiöld, Linker histone defines structure and self-association behaviour of the 177 bp human chromatosome. Sci Rep 11, 380 (2021)) of canonical chromatosomes with H1 bound and two DNA linker arms. The STA map clearly showed the secondary structure features of all core histones, the major and minor groove of the wrapped DNA emerging into the DNA linkers, and the histone tails of histone H3 and H4 projecting outwards through the DNA. Without imposed 2-fold symmetry the resolution was slightly reduced (Fig S1,S3, Table S1), but the asymmetrically associated histone H1 more clearly resolved. Extensive classification attempts also identified nucleosomes in which H1 was shifted to either DNA linker, characteristic for off-dyad binding (Fig S4). These data suggest that an H1 histone variant, which interacts with both the linker DNA arms and the DNA superhelix at the dyad, plays a role in restraining the positioning of linker DNA. This regulation influences the arrangement of nucleosomes relative to one another and is particularly abundant at the nuclear periphery in resting T cells. In addition, to the best of our knowledge, our nucleosome structure from resting T cells represents the smallest non-oligomeric structure resolved to secondary structure resolution by STA in situ to date, with a molecular weight of about 250 kDa.

### Determining DNA linkers between nucleosomes

To analyze multi-nucleosome arrangements in higher detail consecutive nucleosomes were connected by tracing the DNA linkers. First, a distance-based clustering as implemented in cryoCAT ( B. Turoňová, cryoCAT [software]. (available at https://doi.org/10.5281/zenodo.13916774)) was used to identify the candidate nucleosomes to which they could be connected by a DNA linker. For this, neighbors that have at least one of their linker arms (linker 1 and linker 2 in Figure 3b) within 20 nm of at least one of the two linker arms of the other nucleosome were selected. Within a given local cluster defined by this distance criterion, the positions and orientations allowed to connect candidate nucleosome pairs by using a physics-based DNA model of the bending energy *U*_{bend} of the DNA linker (Fig. 2a and Fig. 3a). This bending energy *U*_{bend} is calculated based on the linker length (L), the bending angle (θ), and the configuration of the DNA linker between a pair of nucleosomes that is connected (where Γ denotes the specific linker connection in Fig. 3b) (see Methods for details) as follows: ${U}_{bend} \left(θ, L\right) = \frac{2 {l}_{p}}{L} {\left(\frac{θ}{2}\right)}^{2} {k}_{B} T$

Here *lₚ* ≈ 50 nm is the B-DNA persistence length at physiological conditions, *k_{B}T* is the thermal energy and *L = θr* is the length of the arc of a circle sector of radius r and central angle θ (in radians). The radius r is related to the Euclidian distance *D* between the ends of the linker arms on the two nucleosomes through $r = D / \left(2 sin\left(θ/2\right)\right)$

With the energy function in equation 1, the probability that the DNA linker arms of nearby nucleosomes are connected by a DNA linker was estimated as $P \left(L, θ\right) = {e}^{- \frac{L}{{L}_{0}}} {e}^{- \frac{{U}_{bend} \left(θ, L\right)}{{k}_{B} T}}$ where *L*₀ is a reference linker length, which we set to *L*₀ = 15 nm as an expected typical linker length. The resulting probability function thus penalizes very long but straight as well as very short but strongly bent linkers. Both *L*₀ and *lₚ* are parameters of the algorithm that can be adjusted according to species, tissue, cell type, cell state, and genome location, if needed.

In a spatial cluster of *N* nucleosomes, in this way the relative probabilities for each of the N× *N* × 4 possible connections to be formed were estimated, where the factor of 4 accounts for the four possible connections of the two arms on each nucleosome. The resulting connectivity "matrix" *Pᵢⱼₖ = P(Lᵢⱼₖ, θᵢⱼₖ*) of size N× *N* × 4 accounts for all possible connections in a probabilistic manner, where *i and j* index the *N* nucleosomes of the cluster and k indexes the four possible linker-arm connections between two nucleosomes. Connections are formed in a step-wise manner whereby the most likely connections are assigned in a "greedy" algorithm. This algorithm has three steps that are applied iteratively:
1. Identify the highest value in the current *Pᵢⱼₖ* matrix, which gives a connection (*n, m, l*) between nucleosomes n and *m* for linker connection *l*
2. If the probability exceeds a predefined threshold, *Pₙₘₗ* > *Pₘᵢₙ*, add (*n, m, l*) to the list of connections; otherwise stop the search
3. If (*n, m, l*) has been added as new connection, update the connectivity matrix by setting *Pₙₘₗ* to zero; in addition, we set to zero all entries in the *Pᵢⱼₖ* matrix that lead to an unfeasible solution with a closed form of the DNA (i.e., with the DNA emanating from one nucleosome arm and eventually returning to the other arm of the same nucleosome if traced along the list of connections) or to multiple connections to the same linker arm
4. Return to step 1

Results for the matrix *Pᵢⱼₖ = P*(*Lᵢⱼₖ, θᵢⱼₖ*) of relative connection probabilities between nucleosome arms are visually illustrated in Fig. 2b and FIG. 3c. Shown is the *Pᵢⱼ =* ${max}_{k} {P}_{ijk}$ . It was found that multiple nucleosomes can be confidently linked by choosing pairs connected with high probability. Similar probabilities for multiple possible pathways rarely arise, leaving the linker solution clear in the vast majority of assigned nucleosome pairs (Fig. 4). Therefore, a simple "greedy" algorithm sufficed here to connect nucleosomes, starting with the pair of nucleosome arms connected with the highest probability to the not-yet-connected pair with the next-highest probability and so on, always ensuring that no closed DNA loops are formed. The algorithm stops if the next connection falls below a preset threshold, here set to *Pₘᵢₙ* = 0.1. Although most nucleosome connections are pairs (Fig. 5), in some cases, up to kbp-long chromatin fragments become interconnected (Figs. 3c,d, and Fig. 5), and ultimately break as the chain of nucleosomes reaches the edge of the FIB lamella.

To validate the outcome of the algorithm, i.e. the assigned nucleosome pairs, we subjected the predicted DNA linkers to STA. The averages clearly showed elongated density with the expected width underscoring that we had identified regions that contained DNA (Fig 4). We next grouped the subtomograms according to the predicted linker length, and subjected the individual classes to STA separately. We found that the length observed in the averages corresponds to the linker length predicted by our algorithm (Fig 4). If the validation were less successful, the above algorithm could be refined further. For instance, the energy function can be extended to account for additional known properties of DNA, e.g., of the torsion (twist) in the double helix of ~10.5 basepairs per full turn, where the twist (phase) of the linker ends has to match those of the respective linker arms. In addition, the parameters of the algorithm (*L*₀, *lₚ*, *Pₘᵢₙ*) can be adjusted, e.g., to account for cell type or state. The "greedy" algorithm of connecting linkers can be replaced, e.g., by a probabilistic sampling of nucleosome connections according to the matrix *Pᵢⱼ*. Here, such refinements were not found to be necessary.

### In sillico determination of a number of segments in a nucleosome distance pattern that is sufficient for genomic matching

To analyze the feasibility of matching nucleosome distance patterns from the imaging data onto the respective genome of the, we performed in silico experiments on published genome-wide nucleosome positioning data. We obtained nucleosome peaks with respect to their genomic position from a T-cell lymphoma cell line dataset (iNPS peaks from hsaNuc0000225 in the NucMap database (Zhao et al. Nucleic Acids Res 2019) and calculated the distances between the centers of the peaks along the DNA sequence **(****Fig. 6****).** Several exemplifying, random subsequences consisting of multiple nucleosome distances were matched against the entire genome-wide set of distances. Different length-tolerances ranging from 4-8bp were tested to approximate uncertainties in the estimates of the DNA linker length with the above procedure. We found that with seven connected nucleosomes and a tolerance of at least 5 bp, we obtained unique matches of the nucleosome distance patterns. To check for consistency, we repeated this procedure ten times for different randomly chosen subsequences, and observed each time that only one unique match was detected in the entire genome-wide sequence. In the cryo-ET imaging data, up to eight nucleosomes were connected, and thus up to seven intervening linker distances determined. Therefore, this in silico experiment demonstrates the feasibility of locating the genomic position of a patch of chromatin imaged with in situ cryo-ET.

### Matching of nucleosome distance patterns observed in cryo electron tomograms to genomic data of nucleosome positioning

We next matched experimental nucleosome distance patterns that were observed in our cryo electron tomograms, to the corresponding genome data (see methods). For nucleosome distance patterns with 7 segments obtained from the T cell tomograms with an error tolerance of 6 bp there was only one unique match on one chromosome for one of the directions and no matches for the other direction **(****Fig. 7****).** We therefore conclude, that our method in principle allows for unambiguous assignment of a patch of chromatin inside a tomogram to a specific chromosome and even its genomic position on that chromosome. We furthermore note that multiple nucleosome distance patterns from a single tomogram are consistent with each other, in the sense that their matches can be explained with neighboring genomic sequences on the same chromosome **(****Fig 7****.)**

## Claims

1. A method for identifying the linkages between nucleosomes by DNA linkers in a cluster of nucleosomes comprising:
(i) determining the probability of DNA linkers being present between nucleosomes comprising calculating the bending energy of hypothetical linkages between pairs of nucleosomes using position and orientation data of the nucleosomes,
whereby a lower bending energy is indicating a higher probability.

2. The method according to any of the preceding claims, wherein the orientation data of a nucleosome in the cluster is defined by the spatial orientation of its DNA linker arms.

3. The method according to any of the preceding claims, wherein the bending energy of a hypothetical linkage between a pair of nucleosomes is calculated and/or the probability of DNA linkers being present between nucleosomes is determined on the basis of a physics-based DNA model.

4. The method according to any of the preceding claims, wherein for calculating the bending energy of hypothetical linkages between pairs of nucleosomes and/or for determining the probability of DNA linkers being present between nucleosomes the DNA is described with the worm like chain (WLC) model.

5. The method according to any of the preceding claims, wherein the bending energy of a hypothetical linkage between a pair of nucleosomes is calculated as a function of the DNA linker length (L) and the DNA linker bending angle (θ) between the pair of nucleosomes of the hypothetical linkage, preferably between the DNA linker arms on the pair of nucleosomes of the hypothetical linkage.

6. The method according to any of the preceding claims, wherein the bending energy of a hypothetical linkage between a pair of nucleosomes is calculated according to the following formula (1) ${U}_{bend} \left(θ, L\right) = \frac{2 {l}_{p}}{L} {\left(\frac{θ}{2}\right)}^{2} {k}_{B} T$ wherein(L)is the DNA linker length and (θ) is the DNA linker bending angle between the pair of nucleosomes of the hypothetical linkage, *lₚ* is the B-DNA persistence length at physiological conditions and *k_{B}T* is the thermal energy.

7. The method according to any of the preceding claims, wherein the probability of a DNA linker being present between a pair of nucleosomes is determined comprising calculating the bending energy of the hypothetical linkage between the pair of nucleosomes, and preferably by further determining the DNA linker length (L) between the pair of nucleosomes of the hypothetical linkage.

8. The method according to any of the preceding claims, wherein the probability of a DNA linker being present between a pair of nucleosomes is determined according to the following formula (4) $P \left(L, θ\right) = {e}^{- \frac{L}{{L}_{0}}} {e}^{- \frac{{U}_{bend} \left(θ, L\right)}{{k}_{B} T}}$ wherein *L*0 is a reference linker length, (L) is the DNA linker length between the pair of nucleosomes of the hypothetical linkage and *k_{B}T* is the thermal energy.

9. The method according to any of the preceding claims, further comprising
(ii) defining that DNA linkers are present between pairs of nucleosomes for which the highest probabilities result.

10. The method according to any of the preceding claims, wherein step (ii) further comprises a step of determining whether DNA linkers for which the highest probabilities result are technically sensible.

11. The method according to any of the preceding claims, wherein the bending energy of a hypothetical DNA linkage between a pair of nucleosomes is calculated in step (i) for each of the four hypothetical DNA linkages between the four DNA linker arms on the pair of nucleosomes and/or the probability of a DNA linker being present between a pair of nucleosomes is determined in step (i) for each of the four hypothetical DNA linkages between the four DNA linker arms on the pair of nucleosomes.

12. The method according to any of the preceding claims, wherein a cluster of nucleosomes comprises nucleosomes having a predefined distance to each other, preferably of equal to or more than 1 nm to equal to or less than 1000 nm.

13. A method for identifying the path of DNA along nucleosomes comprising the steps of:
(i) identifying the linkages between nucleosomes by DNA linkers in one or more clusters of nucleosomes according to the method of any of the preceding claims,
(ii) identifying consecutively connected nucleosomes, thereby identifying the path of DNA along nucleosomes.

14. A method for determining the nucleosome distance pattern of a patch of chromatin comprising the steps of:
(i) identifying the path of DNA along nucleosomes, preferably in an image,
(ii) calculating the lengths of the DNA of the pairs of nucleosomes of the path, wherein the length of the DNA of a pair of nucleosomes comprises the DNA linker length (L) and the length of the DNA wound up on one nucleosome.
wherein the lengths of DNA of the pairs of nucleosomes of the path defines the nucleosome distance pattern of the patch of chromatin.

15. A method for identifying the genomic position of a patch of chromatin comprising the steps of:
(i) determining the nucleosome distance pattern of a patch of chromatin according to the method of claim 14,
(ii) determining matches between said nucleosome distance pattern of the patch of chromatin (i) and reference nucleosome distance patterns of chromatin obtained from databases thereby identifying the genomic position.

16. A method for determining the nucleotide sequence of **DNA** comprising the steps of:
(i) identifying the genomic position of a patch of chromatin according to the method of claim 15,
thereby obtaining the nucleotide sequence of said genomic position from the database.

17. A computer program comprising instructions, which, when the program is executed by a data processing device, cause said device to carry out the steps of the methods according to any of the preceding claims.

18. A data processing system comprising means for carrying out the steps of the methods according to any the preceding claims.

19. A computer-readable medium comprising instructions which, when executed by a data processing system, cause the data processing system to carry out the steps of the methods according to any of the preceding claims.
